(19) Europäisches Patentamt
European Patent Office
Office européen des brevets

(11) **EP 4 083 223 A1**

(12) **EUROPEAN PATENT APPLICATION**
published in accordance with Art. 153(4) EPC

(43) Date of publication:
**02.11.2022 Bulletin 2022/44**

(21) Application number: 20905211.7

(22) Date of filing: **25.12.2020**

(51) International Patent Classification (IPC):
*C12Q 1/68* (2018.01)          *G01N 33/483* (2006.01)
*G01N 33/53* (2006.01)          *G01N 33/543* (2006.01)
*G01N 21/64* (2006.01)

(52) Cooperative Patent Classification (CPC):
**C12Q 1/68; G01N 21/64; G01N 33/483;
G01N 33/53; G01N 33/543**

(86) International application number:
**PCT/JP2020/048764**

(87) International publication number:
**WO 2021/132576 (01.07.2021 Gazette 2021/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.12.2019  JP 2019234330
20.11.2020  JP 2020204585**

(71) Applicant: **MYTECH Co., Ltd.
Himeji-shi, Hyogo 672-8004 (JP)**

(72) Inventors:
• **HASEGAWA, Yuki
  Himeji-shi, Hyogo 672-8004 (JP)**
• **HASEGAWA, Katsuyuki
  Himeji-shi, Hyogo 672-8004 (JP)**

(74) Representative: **Algemeen Octrooi- en
Merkenbureau B.V.
P.O. Box 645
5600 AP Eindhoven (NL)**

(54) **AUTOFLUORESCENCE-BASED LIQUID BIOPSY METHOD TARGETING NUCLEOSOMES FRAGMENTED BY APOPTOSIS**

(57)     [Problem] Provided is a method for detecting of an autofluorescence Liquid Biopsy of Methylated Fragmented DNA (fragmented nucleosome) released into the blood by cell apoptosis as a disease-related substance

[Solution] The inventive method comprises a) a step of capturing the fragmented DNA (fragmented nucleosome) in the analyte as a disease-related substance onto the plasmonic metal meso-crystals ; b) a step of irradiating the captured fragmented DNA (fragmented nucleosome) on the plasmonic metal meso-crystal with excitation light to enhance the autofluorescence by the surface plasmon enhancing effect, and acquiring a fluorescent colony image via a filter in a longer wavelength range than the excitation light filter; c) a step of adopting a pixel that exhibits a brightness greater than or equal to a predetermined threshold value of said fluorescent colony image; d) calculating a ratio of a total area value of pixels greater than or equal to a predetermined threshold value of a different two-wavelength region of the adopted measurement region.

**EP 4 083 223 A1**

Fig. 10

Liquid Biopsy Identification

B/G ratio

Healthy person    Benign tumor    Malignancy

**Description**

TECHNICAL FIELD

The present invention relates to a liquid biopsy method based on autofluorescence signal of nucleosomes as cancer-based disease information, wherein the target to be detected or measured is nucleosomes to be fragmented and released from cells into the blood or the body liquid caused by cell apoptosis and the targets to be detected or measured are excited by surface plasmon enhancement, and the autofluorescence can be measured as the epigenetic information.

BACKGROUND OF THE INVENTION

(Importance of Epigenetics)

In the apoptotic cells, inhibitors of CAD (caspase-activated DNase) are degraded, and the DNA can be cleaved into nucleosome units by the activated CAD, so that the DNA is said to be fragmented as a multiple of approximately 200bp (Non-Patent Document 5). This fragmented DNA (fragmented nucleosome) contains both genetic and epigenetic information carried by genomic DNA. That is, although the genetic information carried by the genomic DNA is in the base sequence, it may indicate different information even if the base sequence is the same. In other words, the DNA in our cells exists in a form wrapped around histone proteins, and the chemical modifications such as acetylation of histone proteins alter the DNA function. The inheritance of such changes is called as Epigenetics. The different cells make up an individual different function despite having the same genomic DNA which is received from the same fertilized egg, and their function are different each other because their cellular epigenetics are different. The epigenetic information changes the structure of DNA by adding or deleting molecules, and switches on or off the expression of genes in genomic DNA. In other words, the epigenetics of each cell is important for the proper functioning of the various individual cells (reported by Professor of Tohoku University, Dr.Takahiro Arima, in "Human Epigenetics and Evolution"). Thus, it is said that most naturally occurring cancer cells have a lot of DNA methylation and chromatin abnormalities. In the cancer cells, it is well known that a reduced genome-wide DNA methylation increases chromosomal instability and promoter hypermethylation suppresses expression of tumor suppressor genes. Although the genome-wide hypomethylation and hypermethylation of tumor suppressor are seemingly the opposite phenomenon, it is suggested that genomic regions rich in repeat sequences, such as transposons, and promoter regions of genes are different in the control mechanism of DNA methylation. Also, nuclear structural abnormalities (nuclear atypia) are known as common features in cancer cells, but they have been speculated to reflect changes in chromatin due to genome-wide hypomethylation. Thus, epigenetic regulatory abnormalities in cancer process can be understood as one of the characteristics common to cancer cells. Professor Dr. Mitsuyoshi Nakao, therefore, at Institute of Genetic Medicine, Kumamoto University, has tried to advance the analysis of epigenetic abnormalities of cancer, focusing on 1) MBD1 and zinc finger proteins involved in inactivation of hypermethylated genes, 2) MCAF1 that is highly expressed in cancer cells to alter gene regulation and 3) HMCA1 and HMGA2 chromatin factors related to malignant traits of cancer cells, so that it is understood that it is essential to analyze the epigenetics of individual cells.

(General Biopsy)

The diagnosis of cancer is usually made by biopsy, in which a portion of the cancer tissue is removed by puncture, endoscopic processing, and histopathology of the tissue pieces is performed. Many biopsies (biopsy), they are invasive procedures that involves the risk of bleeding and complications of bacterial infections, and sometimes the biopsy may be difficult in some major areas. To overcome this problem, attempts to diagnose disease with an accuracy comparable to pathologic diagnosis using easily available specimens, such as blood and urine, are called liquid biopsies, and are attracting great attention as being suitable for clinical applications because of their rapidity and simplicity. In addition to circulating tumor cells (circulating tumor cells: CTCs), peripheral circulating tumor DNA (circulating tumor DNA: ct DNA), Exosomes and peripheral blood Circulating microRNAs (miRNA) and the like can be listed in the case of the cancer.

Here, circulating tumor cells (CTCs) are cells that liberate from primary or metastatic tumor tissue into the blood via epithelial-mesenchymal transition (EMT) and circulate in the bloodstream. After release from the primary tumor site, CTCs circulate in the blood and invade other organs to form metastatic tumors (metastases). Since this CTC is present in the peripheral blood of cancer patients, it is expected that detecting this will allow us to judge the process of metastasis and help predict the prognosis of treatment. However, compared with other blood cells, there is a disadvantage that only a very small amount of CTC is present, so the detection is very difficult. On the other hand, the peripheral blood circulation microRNAs (miRNA) and Exosomes containing them are expected to be particularly useful for early diagnostic purposes.

This is because microRNAs play key roles in various life phenomena as fine tuners of gene expression, which bind complementarily to messenger RNA (mRNA) prior to translation into proteins in cells and inhibit translation of mRNA, and it is known that in cancer cells, expression control mechanisms of microRNAs are broken down, and for example, microRNAs that promote cell proliferation are highly expressed. Nearly all cells have secreted extracellular vesicles (Exosomes) in recent years, which makes it possible to diagnose early-stage cancer by detecting Exosomes secreted by early-stage cancer cells in body fluids. Therefore, since it is desired to specifically detect Exosomes derived from diseased cells even when exosomes are analyzed, recovery by ultracentrifugation takes time, and high throughput is difficult. In addition, this type of liquid biopsy method, including the diagnostic technique using circulating neoplastic cells (CTCs, Circulating Tumor Cells), requires hemolysis of erythrocytes from a sample of cancer patients (4 ml of peripheral blood), elimination of blood cells using CD45 antibody beads, and sorting of cytokeratin-positive and CD45 negative cell fractions, which limits the provision of the liquid biopsy method in clinical applications that can be analyzed in a short period of time (Non-Patent Documents 1, 2, and 3).

Therefore, in order to analyze epigenetic information of each cell easily, the present inventors intentionally researched and found that CAD (caspase activity) is active in apoptotic cells. Inhibitors of the sexualization DNase) are degraded, and the activated CADs cleave the DNA in nucleosome units, so they are fragmented as approximately 200-bp-fold more DNA. This post-apoptotic fragmented DNA (fragmented nucleosomes) contains both genetic and epigenetic information carried by genomic DNA and when histone proteins undergo chemical modifications, such as acetylation, the DNA itself undergoes modifications, such as methylation, which alters its behavior.

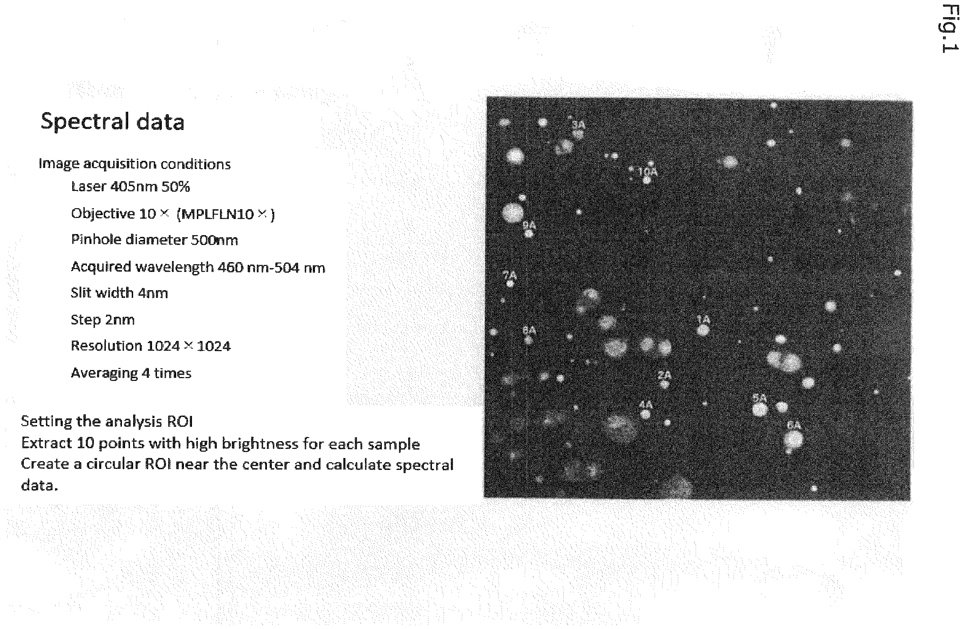

The present inventors have found that such fragmented DNA (fragmented nucleosome) exists in a form (fragmented nucleosome) wrapped around a histone protein after fragmentation, is methylated, has a positive charge, is selectively adsorbed and collected on the surface of a plasmon metal meso-crystal exhibiting a negative charge in blood, and when enhanced by a surface plasmon enhancement effect, such fragmented DNA (fragmented nucleosome) is observable by emitting autofluorescence having a brightness equal to or higher than a predetermined brightness which can be confirmed by a fluorescence microscope (FIG. 1). It has been difficult to selectively collect methylated nucleosomes, even though cancer suppressor genes, including p53, are methylated by epigenetics, and although the methylated nucleosomes to be detected is essential as cancer disease information. In addition, the fragmented DNA captured from blood and so on after cell apoptosis, i.e., fragmented nucleosomes, can aggregate somewhat on the biochip, and the autofluorescence colonies are observed as multiple colonies like night sky constellations, and small ones, about 25μm and large ones, about 150μm were observed. It is very surprising phenomenon.

(Recognition of autofluorescence)

Previously, in cell observation, the autofluorescence generally became background light, and the signal-to-noise (S/N) ratio of the fluorescence image is reduced. Therefore, in the endoscopy, a method of fluorescently labeling the target has been recommended. Therefore, in order to obtain the best data from the fluorescence image, it is necessary to make the difference between the signal (S) and the background (N) as large as possible (Non-Patent Document 6). Accordingly, in a process of cell observation by fluorescence microscopy using autofluorescence, another parameter of fluorescence, lifetime can be used in spite of fluorescence intensity of autofluorescence (Non-Patent Document 7)

Prior art of the documents

Patent Document 1: WO2015/170711 Publication
(The detection of the autofluorescence of nucleosomes and the histogram of the reflection light of the captured cancer related substances)
Patent Document 2: JP 2011-158369 Publication
Patent Document 3: WO2013/039180 Publication

Non-Patent Document 1: Detecting Peripheral Blood Circulation Tumor Cells (CTCs): Circulating tumor cell isolation and diagnostics: toward routine clinical use. Cancer Res 2011; 71:5955-60.
Non-Patent Document 2: Gorges TM, Pantel K: Circulating tumor cells as therapy-Related biomarkers in cancer patients. Cancer Immunol Immunother 2013;62: 931-939.
Non-Patent Document 3: Permuth-Wey J et al., A Genome-Wide Investigation of MicroRNA Expression Identifies Biologically-Meaningful MicroRNAs That Distinguish between High-Risk and Low-Risk Intraductal Papillary Mucino us Neoplasms of the Pancreas., PLoS One., 2015;10: e0116869.
Non-Patent Document 4: Ellen Heitzer, Peter Ulz and Jochen B. Geigl, Circulating Tumor DNA as a Liquid Biopsy for Cancer., Clinical Chemistry 2015; 61:112-123

Non-Patent Document 5: Cell,2016 January 14;164: Cell-free D NA comprises an in vivo nucleosome footprint that informs its tissues-of-origin

Non-Patent Document 6: Current Status of Self-Fluorescent Endoscopes Vol.58(4) Apr.2016

Non-Patent Document 7: Biophysics 53(3), 166-169(2013)

SUMMARY OF THE INVENTION

Problems to be solved by the Invention

As a result of intensive research, the present inventors focused on the deep association with disease (for example, Dr. Yoshihiro Sowa, and Dr. Toshiyuki Sakai reported in "cancer and epigenetics" that: 1) carcinogenesis is a genetic disease caused by abnormalities in oncogenes and onco-suppressor genes, as widely accepted, but also 2) carcinogenesis is caused by epigenetic abnormalities which is caused by modifications to bases, especially by methylation. It has been clarified that quantitative abnormalities caused by hypermethylation as well as qualitative abnormalities of oncogenes are important for the carcinogenesis mechanism, when fragmented DNA (fragmented nucleosomes) released by pathological cell death typified by apoptosis in blood or other body fluids as a result of the occurrence of disease is the subject of investigation. The inactivation of p14 and p53 as well as the hypermethylated RB gene, and p16, is explained as an inactivation of the cancer suppressor gene, which undergoes hypermethylation in cancer cells, so that the carcinogenic mechanism has been very much attractive.).

Therefore, it is important for cancer screening to selectively capture fragmented DNA (fragmented nucleosomes) containing the hypermethylated cancer suppressor gene and quantitative abnormalities thereof. In addition, since a plurality of colonies are observed in the fluorescence image as in the constellation in the night sky shown in FIG. 1. It is very surprising that, the autofluorescence has a luminance of a predetermined value or more and can be confirmed by a fluorescence microscope when the enhancement is performed by the surface plasmon enhancement effect of the plasmon metal meso-crystal (FIG. 1), and when pixels exhibiting a luminance of a predetermined value or more are selected and analyzed, a result with a high correct diagnosis rate can be obtained. That is, the problem to be solved by the present invention is to target fragmented DNA (fragmented nucleosome) released into the blood and so on by cell apoptosis by histone modification analysis and chromatin structure analysis using a tissue sample.

It is an object of the present invention to provide a method for detecting and diagnosing a tumor leading to the development of various diseases, particularly cancer, by a liquid biopsy method using autofluorescence of fragmented DNA (fragmented nucleosome) as a disease-related substance.

Means for Solving the Problem

The present invention, which comprises:

a) charge capturing fragmented DNAs (fragmented nucleosomes) in the sample as a disease- related substance on a plasmonic metal meso-crystal by bringing a solution containing a body fluid to contact to a plasmonic metal meso-crystal substrate;

b) irradiating the captured fragmented DNAs (fragmented nucleosomes) on the plasmonic metal meso-crystal with 1) a single wavelength exciting light as a laser light source or 2) a constant wavelength width light acquired through a filter from a LED light source, thereby enhancing the autofluorescence of the captured disease-related substance colony by the surface plasmon enhancing effect, and determining a fluorescence image of the fragmented DNAs (fragmented nucleosomes), and acquiring a fluorescent colony image via a filter having a longer wavelength range than the excitation light filter:

c) adopting a pixel that exhibits a brightness greater than or equal to a predetermined threshold of said fluorescent colony image:

d) calculating 1) a total area value of pixels above a predetermined threshold in a predetermined wavelength region of an adopted measurement region, or 2) a ratio of total area value of pixels above a predetermined threshold in two different wavelength regions of an adopted measurement region.

In the present invention, a total area value and the ratio of the total area values of pixels are important, so that the excitation light should have 1) a constant wavelength width of the excitation light for acquiring through filters the excitation light from the excitation light or LED light source and 2) a single wavelength by a laser light source. In adopting the region of the predetermined luminance from the observed fluorescent colony, ROI is adopted. The reason why pixels having a brightness equal to or higher than a predetermined threshold value in the fluorescent colony, are selected, is to improve the measurement accuracy. Further, in the present invention, in the calculation step, the pixels of the fluorescent colonies having a predetermined luminance or more, which are binarized and selected, and the total area thereof is calculated. Or the ratio of the total areas of the two different wavelength regions are calculated. The pixels having a predetermined brightness or more tends to respond to epigenetic information. The fragmented DNA (fragmented nucleosome) contains both genetic information and epigenetic information carried by genomic DNA, so that only epigenetic information is adopted as much as possible by using a ratio of the two different wavelengths. Therefore, pixels of fluorescent colonies above a threshold are selected and are separated into at least two stages (Yes) or (No) at risk of cancer from their total area value or their ratio of differing two-wavelength regions (ratio value). Preferably, the data are separated into 3 stages where there are 3 stages: one is cancer risk (Yes), the other is necessity of observation, and further more low cancer risk (No). On the other hand, the wavelength range of RGB to be adopted is preferably in the G or B region, and the ratio of G/B or B/G is preferably calculated. This is because, when the spectral spectrum of the fluorescent colony is observed, a peak peculiar to the disease, in particular, a peak peculiar to the cancer is observed in the B or G region.

In addition, in the present invention, as a detection target, a target to be captured is fragmented DNAs (fragmented nucleosomes) of approximately 200bp in length that cleave DNA in nucleosomes by activated CADs (caspase-activated DNase) and contain both genetic and epigenetic information carried by genomic DNA, and they are preferably used for ratio characterization to facilitate the removal of only epigenetic information. This method is a Liquid Biopsy method in which captured fragmented DNAs (fragmented nucleosomes) are excited at excitation wavelengths in the same or different two wavelength regions of the B or G region to obtain fluorescent colonies, which are collected through filters in different two wavelength regions, and the ratio of the total area values of the pixels collected therefrom is the B region/G region or the G region/B region.

The body fluid which is the specimen is plasma or serum separated from lymph fluid or blood, and fragmented DNA (fragmented nucleosome) shows positive charge by binding to histone protein, so that it can be selectively captured. In samples, when it is an iPS cell, it is intended to discriminate canceration of the iPS cell.


EFFECTS OF THE INVENTION

According to the present invention, firstly, a disease-related substance, a multiple of approximately 200bp of fragmented DNA (fragmented nucleosomes) that has broken the DNA on a per-Nucleosome basis by activated CAD (cassPase activation DNase) can be selectively captured. Normally, although autochrome is noisy, but is revealed by the surface-plasmon enhancement effect in the radiological diagnosis, and a luminosity colony greater than or equal to a predetermined threshold is adopted. 1)The total area value of the luminous colony greater than or equal to a predetermined threshold, or 2) the 2-wave ratio correlated with RGB is calculated, thus diagnosing the condition, particularly in the case of cancers, benign cancers, good benign cancers, and healthy patient can be distinguished.

Generally, biological proteins have autofluorescence, but this autofluorescence forms a background of signal (such as autofluorescence, which is not desirable to be fluorescently labeled) in fluorescence observation of a target, resulting in noise. However, since the autofluorescence of this biological protein reflects the structure of the biological protein, according to the present invention, the structural analysis by autofluorescence is particularly effective in the following points. That is, in recent years, from the results of analysis conducted so far, not only genome changes but also epigenome changes tend to accumulate in a large number in the process of development and development of cancer cells, and epigenome modification is roughly classified into chemical control and physical control. Representative of chemical control is the modification of various histone tails called "histone codes". Modifications to tens of amino acids in histone tails have been reported, but chromatin-binding proteins (reader), which recognize these modifications alone or in combinations, play a role in transcriptional control. On the other hand, the primary (nucleosome) and higher-order chromatin structures are considered important for physical control. Since chromatin architecture changes dynamically during normal cellular differentiation and development (chromatin remodeling), and not only is this spatiotemporal control essential, but also dysregulation is involved in diseases such as dysgenesis and cancerization, in histone modification, acetylation (H3K27ac) and methylation of H3 lysine 27, which are characteristically found in regions that positively activate gene expression (enhancer regions), are noticed. Although gene expression states correlate well with chromatin states in promoter regions where transcription origins reside, chromatin states of enhancers are also deeply involved in gene expression regulation unique to the cell lineage. Recently, acetylation (H 3K27ac) enhancers have also been shown to be critical in gene conformation (genomic topologies). Therefore, the observation of autofluorescence using clinical tissue specimens suggests the possibility of early diagnosis of cancer and identification of the cancer site by histone modification analysis and chromatin structure analysis. Thus, the protein conjugate to which the cell's free DNA is bound wraps around the histone to form a nucleosome, which stabilizes the DNA in the nucleosome when the histone protein is methylated. In addition, abnormal DNA methylation suppresses Tumor suppressor gene, resulting in cause of cancer onset, so that it is considered to be responsible for the development of cancer. Therefore, it is an extremely expected target of liquid biopsy to collect and analyze, the cellular free DNA (cf DNA) released from cells into the blood by cell apoptosis, including circulating tumour DNA (ct DNA). However, it is extremely difficult to collect free DNAs (cf NA), including ct NA in blood, and so PCR is the basis of detection because of the trace mount of free DNAs, and the detection is roughly divided into mutation-specific PCR and digital PCR (Non-Patent Document 5). n the present invention, in view of the fact that DNA has a property of being trapped in plasmonic nanometal crystals which usually exhibit a negative charge and strongly binds with a histone protein exhibiting a positive charge, it was attempted to capture this by a chip and detect it by Raman spectral spectroscopy (WO2015/170711). However, the histone protein captured on the chip are cleaved by apoptosis, so it has been extremely difficult to detect Raman light because of extremely small. On the other hand, if the trapped substance is appropriately excited by excitation light, it has been found that if autofluorescence of disease-related substances including free DNA (cf DNA) and fragmented DNA (fragmented nucleosomes) can be manifested by surface plasmon-enhancing effects, the total area value or two-wavelength ratio correlated with RGB, is useful to diagnose disease, in particular, to distinguish cancer patients, benign neoplasm patients, and healthy individuals by using fluorescent colonies having brightness of a predetermined threshold or more as a reference.

Second, according to the present invention, the selective capture of disease-related substances is performed as follows:
Fragmented DNAs (fragmented nucleosomes) that should be targeted include ct DNA Cellular free DNA (cf DNA) is present in the blood as minimal nucleosomes or higher-order chromatin associated with histone proteins. In addition, DNA wrapped around histones is stably present by binding to histone proteins and methylation (According to the document "Cancer and epigenetics" various cancer suppressor genes hypermethylated from cancer cells: hypermethylated RB genes, p16, p14 and p53 are included.) Thus, by providing a chip exhibiting a positive charge, these can be selectively adsorbed or captured. Substances captured on such a chip manifest autofluorescence by surface plasmon effect by specific excitation light, and they are analyzed by fluorescence microscopy, it was found that the fluorescence wavelength can distinguish healthy persons, cancer patients, and other disease patients. In other words, cell-free DNA is also released into the blood from cells of healthy individuals via apoptosis, whereas tumor cell-free DNA is also released from cancer cells, which bind tightly with their own methylation and histone methylation, and release disease-specific fragmented DNA (fragmented nucleosomes) from other diseased cells. DNA released from cells of these healthy individuals, cancer patients, and patients with other diseases has not only genetic aberrations but also epigenetic genetic aberrations due to modifications to bases, and there are chemical or physical differences in the fragmented DNA released from each cell (fragmented nucleosomes). We found that fragmented DNA (fragmented nucleosomes) emitted from cells of healthy individuals, cancer patients, and other disease patients differed in the wavelength of autofluorescence. When the fluorescence wavelength of cancer-related substances collected from cancer patients is classified in more detail, the fluorescence wavelength spectrum or the spectral peak differs depending on the cancer primary site and metastasis site because the epigenome modification is roughly divided into chemical control and physical control, and is affected (according to the spectroscopic spectrum, a slight peak was observed in the fragmented DNA (fragmented nucleosome) of cancer patients at around 515nm). Therefore, it is extremely useful for evaluating various diseases, including cancer, to calculate 1) a total area value of a fluorescent colony of a predetermined threshold or more, or 2) a total area value ratio of a fluorescent colony of a predetermined threshold or more.

Third, DNA released into body fluids, especially plasma or serum, by pathological degradation such as apoptosis of cells combines with histone proteins to form protein conjugates. According to the present invention, autofluorescence of protein conjugates can be manifested by plasmon enhancement and can be observed under a fluorescence microscope to aid in early diagnosis of disease.

This is because abnormalities in protein post-translational modifications are associated with the development of various diseases, but it is possible to perform a simple and rapid proteomic analysis on specimens from patients with various diseases, including cancer, lifestyle-related diseases, and infectious diseases. In this liquid biopsy method, the elucidation of posttranslational modification abnormality associated with the disease is immediately carried out in the clinical field, and not only becomes a diagnostic biomarker for deciding the therapeutic policy and realizing the individualized medical treatment, but also provides a very important guide in the decision of the drug discovery policy for developing the new therapeutic drug. In general, many problems remain to be solved in the analysis of peptides, such as the improvement of the pretreatment technique of the sample and the concentration technique specific to the modifying group, but by utilizing this method, the relationship between the posttranslational modification abnormality and the disease is useful for elucidation.

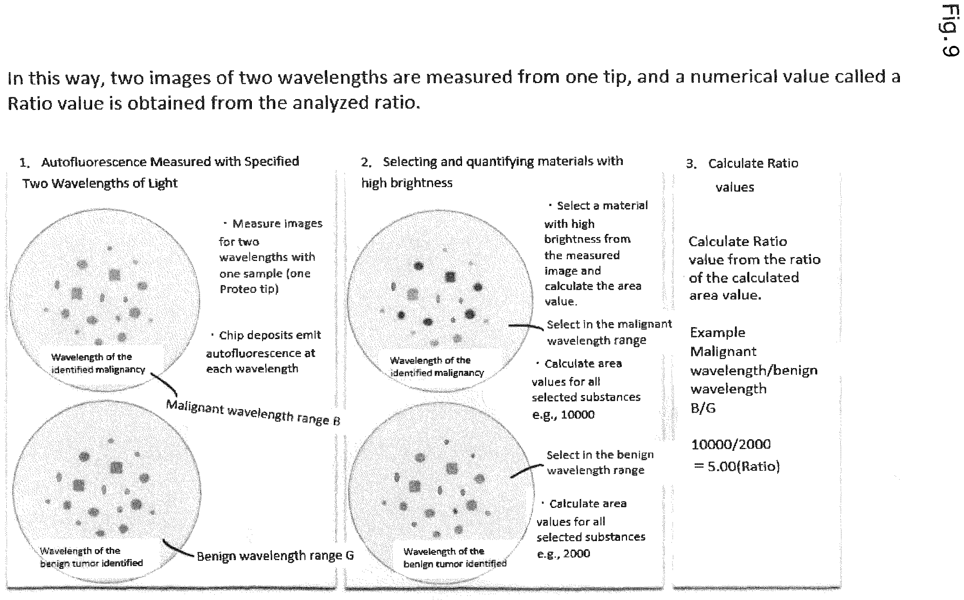

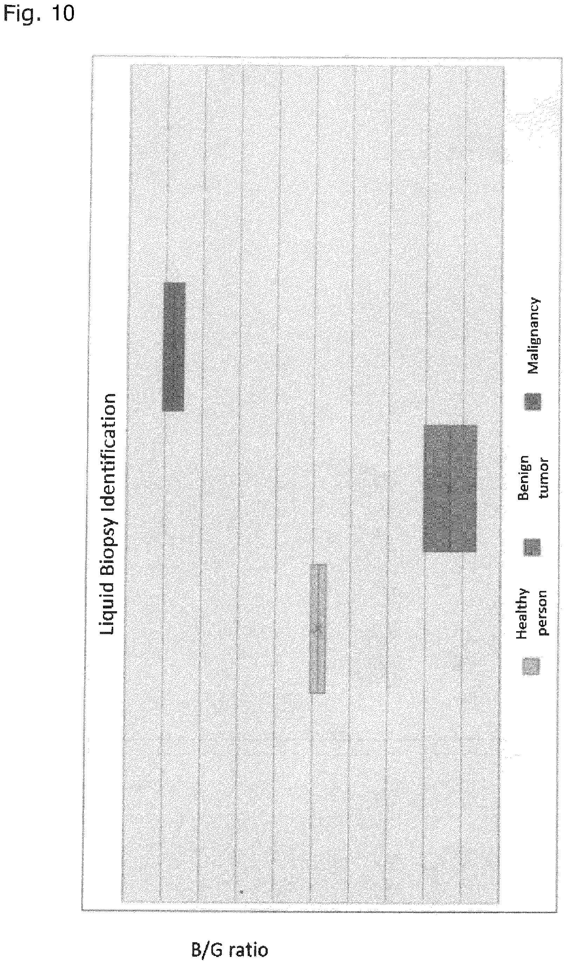

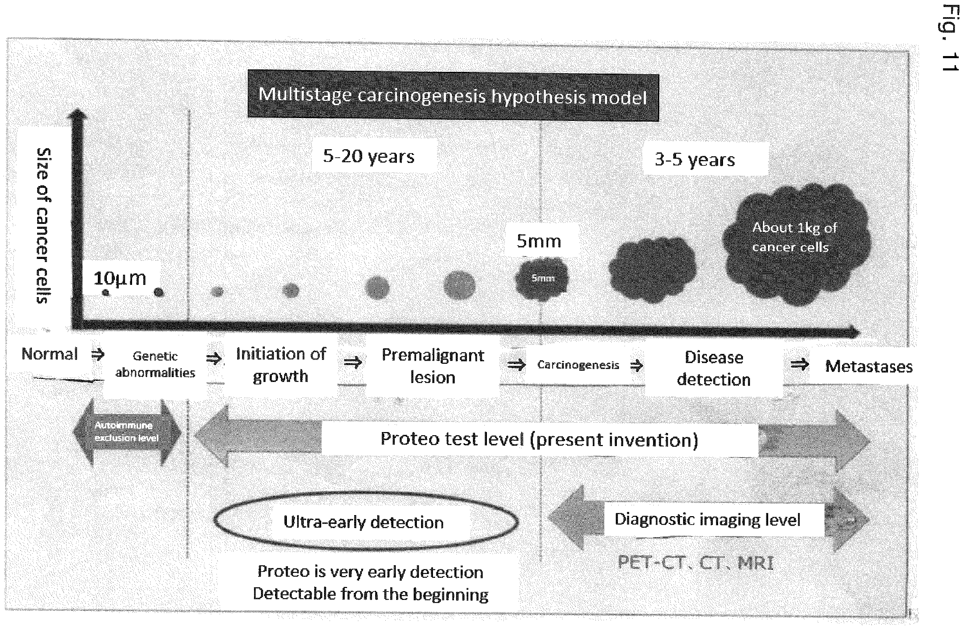

Specifically, according to the present invention, these fragmented DNAs (fragmented nucleosomes) are released into blood or other body fluids by pathological cell death typified by apoptosis by the occurrence of disease, and therefore, are deeply related to the disease. In particular, since autofluorescence in the blue (B) region appears strongly in the malignant tumor and autofluorescence in the green (G) region appears strongly in the benign tumor in the autofluorescence characteristics of the nucleosomes themselves after apoptosis (Fig. 9), it has been found that the area values with high brightness in the blue (B) region and the green (G) region in Ratio values (B/G or G/B) successfully distinguish healthy subjects, benign tumors, and malignancies, and that cancer can be diagnosed in an ultra-early stage, recurrence determination, metastasis determination, and monitoring of the therapeutic effect can be performed (Fig. 10). Because the segmented nucleosomes after apoptosis can detect methylated nucleosomes caused by genetic abnormalities, the present test level (Proteo test level) can realize more faster diagnosis than the present diagnostic imaging level (PET-CT, CT, MRI), as shown in the multistage carcinogenesis hypothesis model shown in (Fig. 11). Based on previous detection of cancer-related substances, an ultra-early detection was found to be possible by the present inventive method.

BRIEF DESCRIPTION OF THE DRAWINGS

FIG. 1 shows a fluorescence image obtained using an Olympus DM (Dichroic Mirror) 405-445/514, where 10 points of high brightness for each sample are adopted.

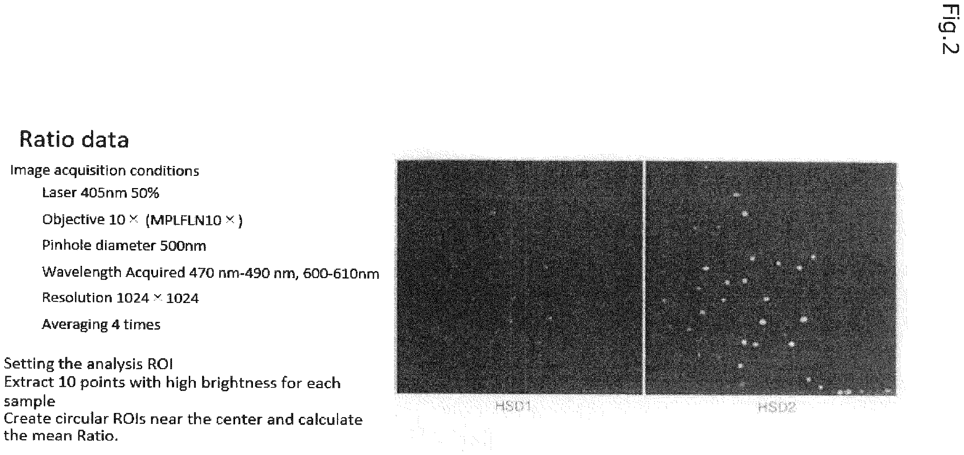

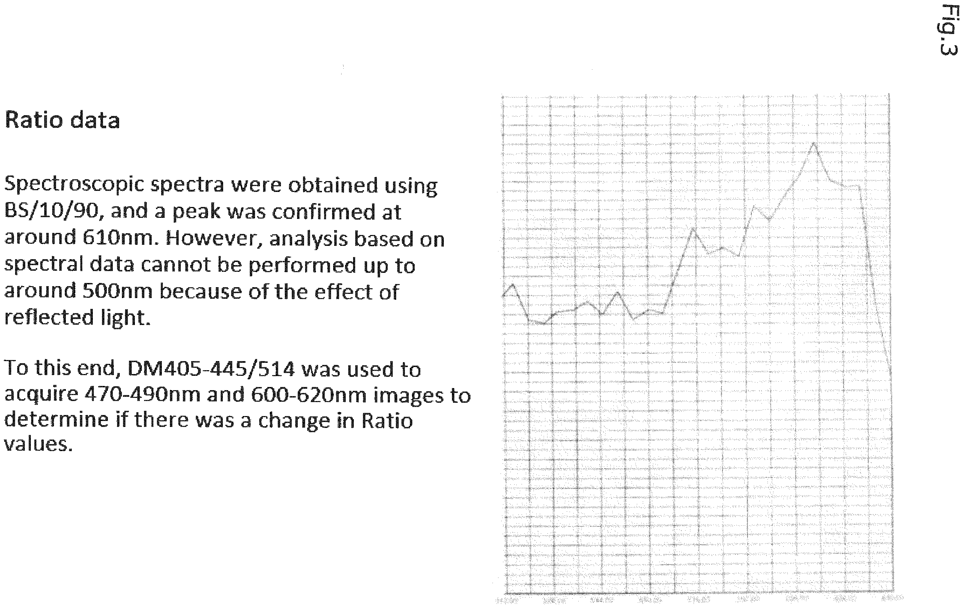

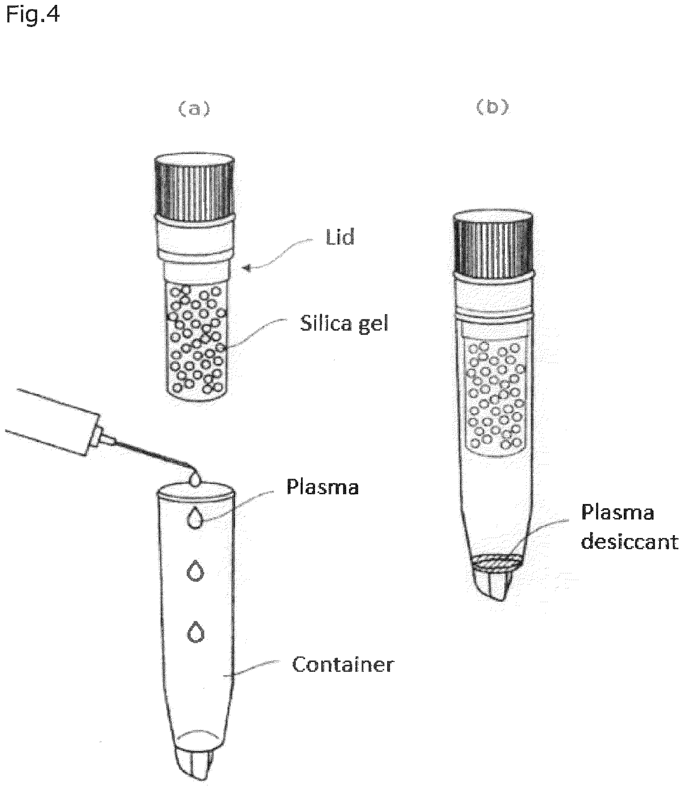

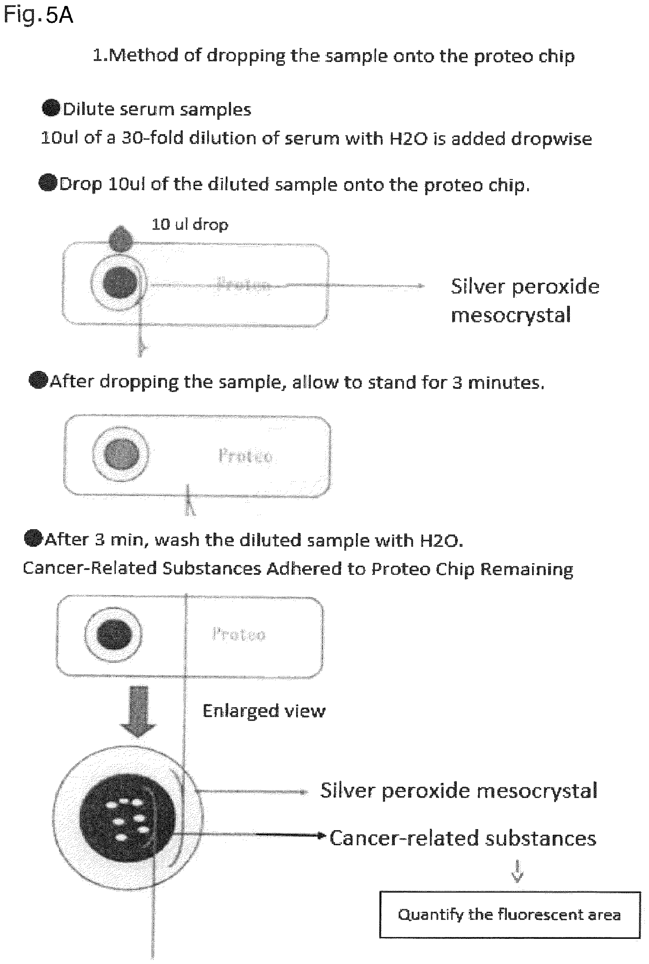

FIG. 2 shows 470-490nm and 600-620nm images acquired using an Olympus DM405-445/514. FIG. 3 is a graph showing spectral spectra obtained using a BS10/90. FIG. 4 shows a silica gel drying vessel, (a) shows a dropping state of a crystal obtained by centrifuging blood, and (b) shows a state in which plasma is dried in a container with silica gel. Fig. 5A shows the first step A of the first method is an explanatory view of the preparation of a measuring chip (proteo chip),

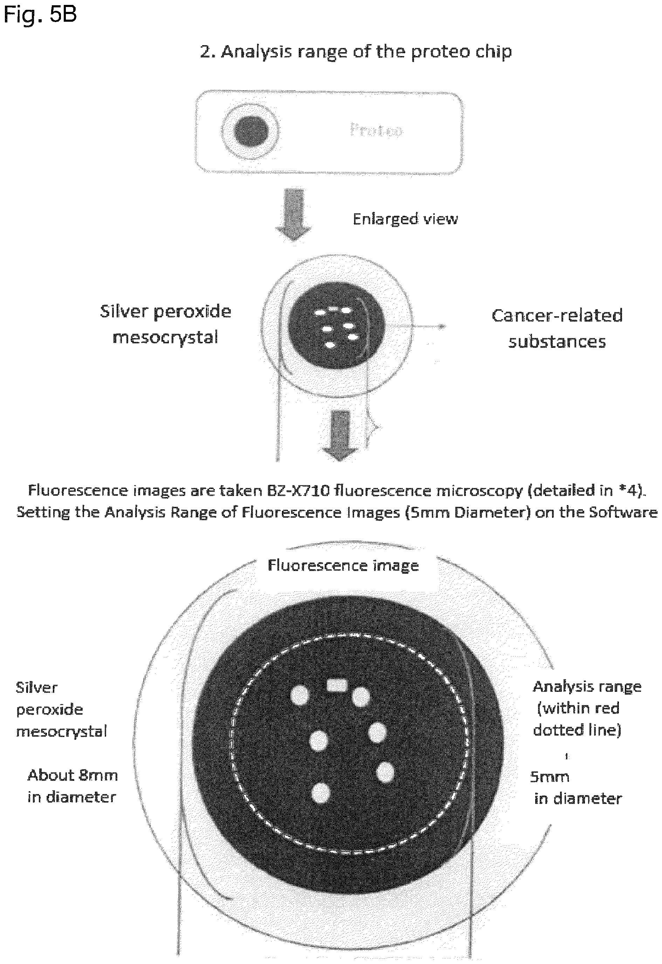

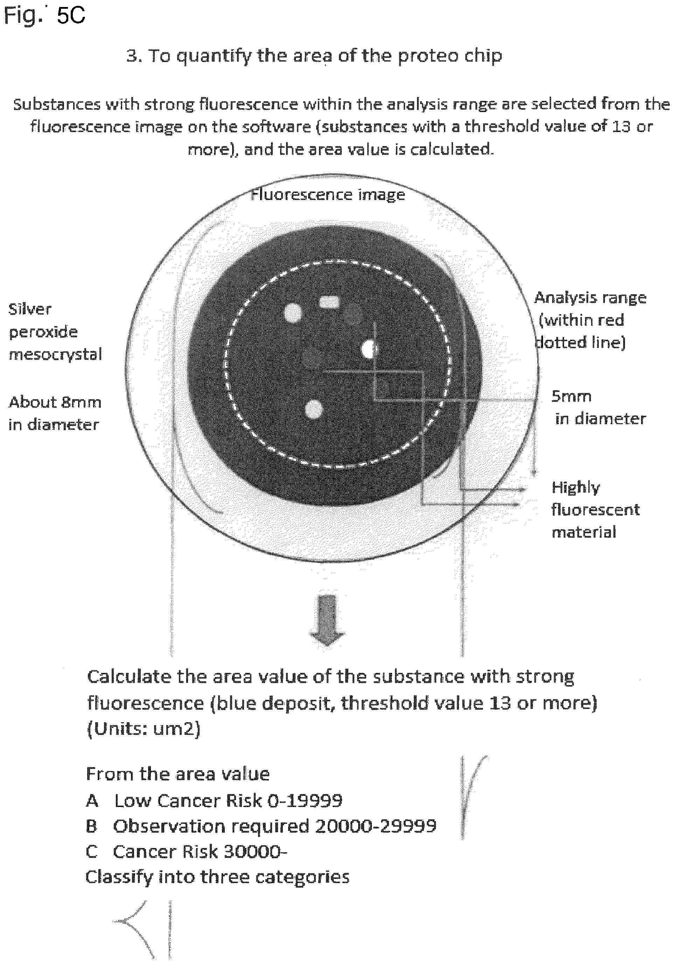

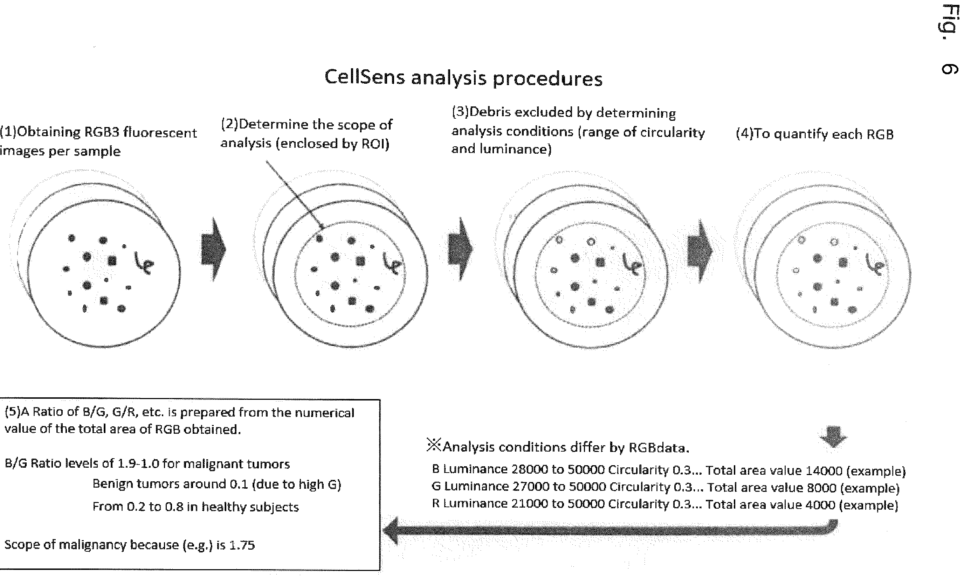

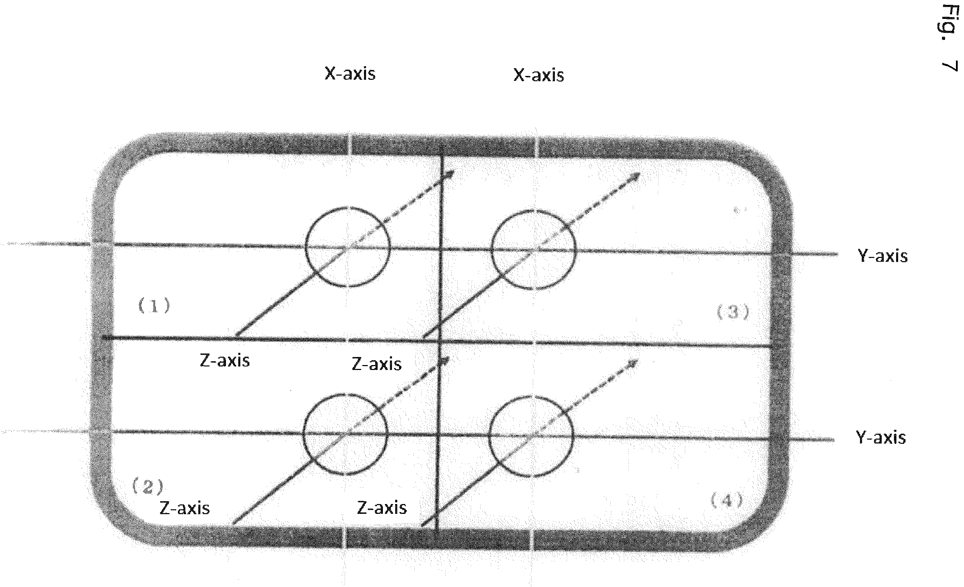

Fig. 5B shows the second Step 2 B of Illustration of the determination of the analytical scope of the proteocip, Fig. 5C show sthe the third Step 3 C of Illustration of the numerical representation of the area of the (adopted fluorescent colony) of the proteocip. Fig.6 shows the image acquiring step of the second method of the present invention, where (1) analysis range determining step, (2)fluorescent colony adopting step, (3) calculation step of the total area value of the selected colonies in OOARGB fluorescent image, (4) is an explanatory diagram showing a step of obtaining RGB total area and (5) preparing a Ratio value such as B/G,G/R from → obtained RGB total area. FIG. 7 is an explanatory view of a microscope stage employed when the method of the present invention is automatically performed;

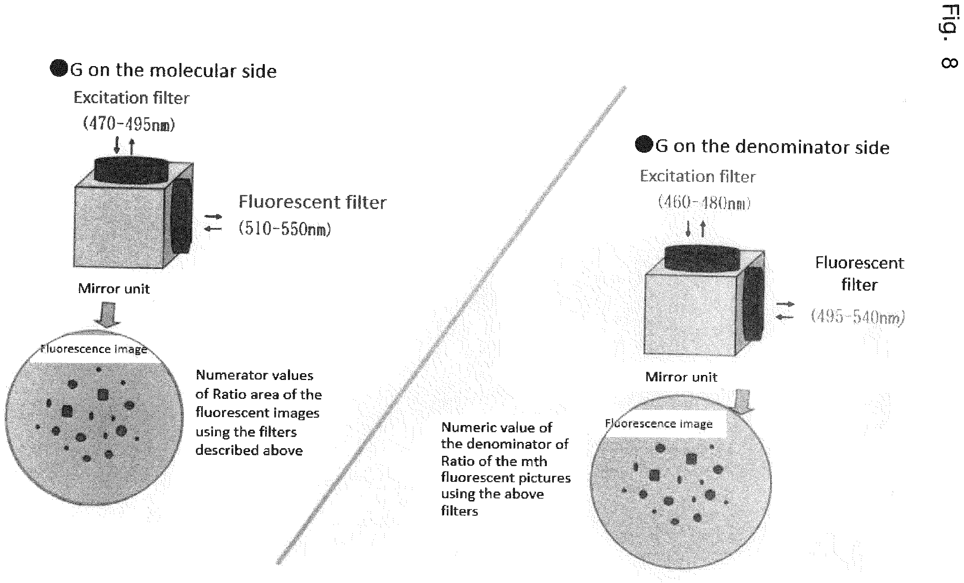

FIG. 8 shows a conceptual diagram of the inventive Ratio calculation process. Fig.9 is an explanatory view of the analysis method when the fragmented nucleosome which exists in the centrifuged supernatant liquid (plasma portion) is measured the autofluorescence using the proteo chip of the present inventi)on. FIG. 10 is a graph showing distributions of Ratio values of healthy subjects, benign tumors, and malignancies that can be identified by the method of FIG. 9, wherein the y-axis height represents the ratio of B region/G region, and the x-axis represents the sample count. FIG. 11 is a comparative illustration of multi-stage carcinogenesis hypothesis models comparing 1) the inventive test levels for detecting fragmented nucleosomes with 2) the test levels by using the current PET-CT, CT , and MRI.

DETAILED DESCRIPTION OF THE INVENTION

In the implementation of the liquid biopsy method according to the present invention, the first method is as follows In the first method, the fragmented DNA (fragmented nucleosome) captured on the substrate is excited by 1) a single wavelength excitation light from a laser or 2) an excitation light of a fixed wavelength width adopted by a filter, and the total area of pixels above a predetermined threshold of the fluorescent colony is measured.

Equipment used: Fluorescence microscopy BZ -X710 made in KEYENCE CORPORATION, light source: Metal halide lamp 80W
Fluorescent Filter: BZ-X Filter DAPI (460±25nm)
Analysis Software: BZ-X Analyzer

a) Selective capture steps for cancer-related agents: as shown in Fig.5A
Measurement substrate having plasmon metal meso-crystalline regions exhibiting surface negative charges in a sample: A proteo chip (Fig. 5A (1)) is contacted with a specimen prepared by diluting a body fluid or a culture solution containing cells as it is or by a diluted culture solution (Fig. 5A (2)), and a protein conjugate exhibiting positive charges in the specimen is trapped by the plasmon metal meso-crystal as a disease-related substance (Fig. 5A (3)).
b) Fluorescence image acquisition process:

(1) Placing the Proteo chip with cancer-related substances on the fluorescence microscope.
(2) Determining the measurement position (X and Y axes) of the chip while viewing the bright-field image, and click the auto focus button to adjust the focus (Z axis, focus) of the chip.
(3) The measurement setting is switched to BZ-X filtering DAPI, and the measurement of the fluorescent images is started. Excitation light is applied to the protein conjugate captured on this plasmon metal meso-crystal (about 8mm in diameter), and autofluorescence of the captured protein conjugate is enhanced by Surface Plasmon Effect.

(4) Step of enhancing by the enhancement effect and acquiring the fluorescent colonies as fluorescent images (Fig. 5B);

c) adoption step of the fluorescent colonies:
Step of binarizing the luminances of fluorescent colonies within the analysis range (5mm in diameter) and selecting fluorescent colonies with luminances equal to or higher than a predetermined threshold
d)Calculation process:
Calculating the total area (Fig. 5C) of fluorescent colonies above the selected thresholds. Here, substances with strong fluorescence (blue deposits, based on luminance values under the following measurement conditions)

Then, a binarization threshold value of 13 or more is adopted, and the area value thereof is calculated (unit $\mu$m2).

It is judged by classifying into three stages: when 0 to 19999 from the area value is A (the gun risk is low), 20000 to 29999 is observation required B and more than 30000 is with gun risk.

FIG. 6 is a second method of calculating a ratio at a two-wavelength from the RGB and/or the related value of RGB from that adopts a fluorescent colony above a certain threshold from a fluorescent image. In FIG. 6 (1), three fluorescence images of RGB are acquired for one sample. Next, an analysis range is determined and surrounded by an ROI (FIG. 6 (2)). Here, "the circularity and luminance of the fluorescent colony" is used as a threshold value, and analysis conditions are determined to exclude dust and the like (FIG. 6 (3)). Thereafter, the total area value of the fluorescent colonies adopted from the fluorescence images of RGB is calculated (Figure 6(4)) The details are as follows.

Typical fluorescence measurements are made using fluorescent dyes, which are observed at the fluorescence wavelengths unique to the dye. The excitation and fluorescence wavelengths are determined by the type of dye. Fluorescence measurements using dyes look at the fluorescence of the dye and are not a measure of the autofluorescence of the substance itself.

On the other hand, the autofluorescence in this invention is thought to be emitted by chemical composition and chemical structure of the substance itself, functional groups, modified compounds, biological structures, etc. In other words, each autofluorescence has an intrinsic fluorescence wavelength. The present invention looks at the intrinsic autofluorescence of cancer-related substances. It is a method to measure wavelengths of autofluorescence inherent to cancer-related substances rather than wavelengths of common dyes from multiple excitation light. We identified the optimal excitation and fluorescence wavelengths for direct measurement of cancer-related substance-specific autofluorescence. From the results of spectroscopic analysis of autofluorescence of cancer-related substances, the wavelengths for measuring the excitation light of emitted RGB and the autofluorescence of RGB were selected and determined. An LED light source was used as the light source, and fluorescence wavelength filters were attached to the Olympus erect microscopy BX-63 to limit the fluorescence wavelength to be measured, and fluorescence images were acquired. Specifically, the excitation light of B is in the range of 375 to 400nm, the fluorescence wavelength is in the range of $460 \pm 25$nm, the excitation light of G is in the range of 470 to 495nm, and the fluorescence wavelength is in the long wavelength range of 510nm or more.The excitation light of R is in the range of 530 to 550nm, and the fluorescence wavelength is in the range of long wavelength of 575nm or more. The B is the excitation light and fluorescence wavelength that the autofluorescence of cancer-related substances while the G is the autofluorescence of benign diseases.

According to the second method, the present invention comprises (1) a step of setting the biochip with the cancer-related substances on the Olympus erect microscopy BX-63, and (2) a step of setting the LED light source and the fluorescent wavelength filter to the setting of B above. (3) focusing (Z-axis) while viewing the real-time fluorescence image of the biochip. When the focus (Z-axis) and measurement position (X and Y-axis) are determined, firstly the fluorescence image of the setting of B is measured. Next, the LED light source and the fluorescence wavelength filter is set to the setting of G, and the focus (Z-axis) and the same measurement position as the previous B setting (X, Y-axis) are adjusted, thereby we can obtain the data of fluorescence images of each of the same cancer-related materials at the same location on the chip can be acquired. The same process is applied to measure the fluorescence image at the same measurement position (X and Y axes) with the LED light source and fluorescence wavelength filter of R setting in the same way.

Analytical Methods

Analysis was performed using imaging software "cellSens" (Nippon Olympus Optics).

(1) Determine the analysis range by surrounding the same cancer-related substance at the same position attached to the biochip of the three kinds of fluorescence images measured by each excitation light with ROI, and calculate the area value of the substance in the set range of RGB below, wherein B is a range to be selected from the range of liminance values 28000 to 50000 and selected to remove deposits having a degree of circularity of 0.3 or less.

G is a range selected from the luminance value from the range of 27000 to 50000, and also selected to remove deposits with a circularity of 0.3 or less. R is a range from the range of luminance values 21000 to 50000 and also selected to remove deposits with a circularity of 0.3 or less.

The luminance or brightness value setting range is selected depending on the fluorescence intensity of the autofluorescence of the adhering substance or the intensity of the light of the LED light source. In addition, the analysis software automatically removes foreign matter having a strange shape by analyzing with a circularity of 0.3, thereby improving the accuracy of the analysis. In the range of 10000 to 70000 and preferably in the range of 20000 to 50000 with a degree of circularity

The range is from 0.9 to 0.1, preferably on the order of about 0.3. This circularity is adjusted according to the context of the fluorescent colony. Ratio values of two-wavelength ratios, such as B/G and G/R, were calculated from the area values of RGB calculated by these measurements and analysis settings. In the case of colon, malignant tumors is ranged from 1.9 to 1.0 in Ratio values of B/G, and benign tumors is around 0.1 for and healthy individuals is ranged from 0. 2 to 0.8. . It ranged from.

By performing fluorescence measurement by dividing the excitation light in this manner, it is possible to obtain a fluorescence image of stronger autofluorescence in each excitation light. By measuring and analyzing the same cancer-related substance at the same position on the biochip with different excitation light and fluorescence wavelengths, more substance-specific autofluorescence can be extracted.

The accuracy of the data could be improved.

FIG. 7 is an image diagram of a microscope stage when four proteo chips (measurement substrates) are set in a holder to automate the measurement. The measurement positions (X-axis and Y-axis) of Tip (1) ~ (4) are decided in advance, and the focuses (z-axis) of the chips (1) to (4) is adjusted by hand. Once the X, Y and Z axes are determined, and RGB of four chips are automatically measured to obtain four RGB fluorescent images.

The body fluid being the analyte is not only plasma or serum separated from the lymph or blood, but also includes urine, saliva, and the like, and the protein conjugate includes cell free DNA (cf DNA) which binds to histone proteins and exhibits positive charges, and the cell free DNA includes circulating tumor DNA (ct DNA) released from the cells. Preferably, the light source that excites the plasmonic metal meso-crystal that captures the disease-related material is used as an excitation light at a wavelength of 405nm that is used to excite the tumor-affinity fluorophore. In this case, since the fluorescence wavelength can be observed around 630nm in that case, it is preferable to focus on the peak wavelength around 630nm as a characteristic of the autofluorescence of the protein conjugate of the cancer-related substance even in the present invention. The protein conjugate captured here is a nucleosome containing circulating tumor DNA (ct DNA) bound to a histone protein or a chromatin with a higher order, and when the histone undergoes methylation modification, the cancer diagnosis of the liquid biopsy method by autofluorescence is performed as a target.

Chip with plasmonic metal meso-crystals exhibiting surface negative charges in the sample

A substrate having a plasmonic metal meso-crystalline region used in the method of the present invention is referred to as a proteo chip made by Mytech. Co. Ltd. The manufacturing method thereof is as follows (see Patent Document 1)

1) An aqueous solution of a metal complex is chemically reduced on a metal substrate having an electrode potential (having a larger ionization tendency) higher than that of the metal forming the complex by an electrode potential difference to aggregate quantum crystals (nano-sized metal complex crystals). In the case of a silver complex, a quantum crystal of the silver complex is formed by aggregating an aqueous silver thiosulfate solution on copper or a copper alloy having an electrode potential (having a large ionization tendency) higher than Ag by a chemical reduction method. Specifically, the concentration of the metal complex in aqueous solution should be determined primarily by considering the size of the quantum crystals to be formed, and when a dispersant is used, its concentration may also be considered, and usually can be used in the range of 100ppm to 5000ppm, but a concentration of 500 to 2000ppm is preferred to prepare a nano-size crystal to be nanoclusters depending on the function of the ligand.

2) The metal complex forming the quantum crystal is selected so as to have a complex stability constant (log $\beta$) or more represented by formula (I) which correlates with the electrode potential E of the supported metal

$$\text{Equation (I): } E° = (RT/|Z|F)\ln(\beta i)$$

wherein E° represents the standard electrode potential, R represents the gas constant, T represents the absolute temperature, Z represents the ionic number, and F represents the Faraday constant.

Here, when the metal complex is a complex of a plasmonic metal selected from Au, Ag, Pt or Pd, it has a localized surface plasmon resonance enhancing effect by the excitation light. Stability constant (formation constant) particularly when the metal complex is a silver complex, it is preferable that (log β i) is formed by the reaction of a silver complexing agent having 8 or more and silver halide, and the silver chloride is preferred as the silver halide, and the complexing agents is preferred to be selected from the group consisting of thiosulfate, thiocyanate, sulfite, thiourea, potassium iodide, thio-salicylate, and thio-cyanurate are preferred as the complexing agent. Silver complex is flat and comprises a quantum dot consisting of nanoclusters with a uniform diameter of 5-20nm, and the size of the quantum crystal becomes 100-200nm.

3) The plasmon metal meso-crystal in the proteo chip used in the present invention is an oxide of the quantum crystal of a plasmon metal complex, which is a negative charge necessary for capturing a methylated nucleosome positively charged in blood by an electric charge, and which exhibits a surface plasmon enhancing effect by irradiation of the excitation light and has an effect of enhancing autofluorescence of the captured methylated nucleosome. In the case of silver complex quantum crystals, alkali treatment (for example, treatment with sodium hypochlorite aqueous solution) seems to form needle-like nanocrystals of silver oxide complex containing silver peroxide as nuclei by the following reaction (as shown in Fig. 5 of Patent Document 1). In addition, the (-) charge is charged in water, while the histone around which the DNA is wound is charged (+).

As shown in (Fig. 7(a) of Patent Document 1), it was found to selectively adsorb positively charged cancer-related substances represented by this free nucleosome. Moreover, the needle-like or acicular nanocrystals of silver oxide containing silver peroxide exhibit surface plasmon enhancement effect by irradiation with the excitation light represented by a laser light and a filtered LED light, and have been found to be preferable for detecting autofluorescence of cancer-related substances represented by adsorbed histones.

$$Na_2S_2O_3+4NaClO+H_2O \rightarrow Na_2SO_4+H_2SO_4(2NaHSO4)+4NaCl$$

$$Ag^++NaCl \rightarrow AgCl + Na^+$$

$$Ag^++3NaOCl \rightarrow 2AgCl + NaC103 + 2Na^+$$

$$Ag^++OH^- \rightarrow AgOH$$

$$2Ag^++2OH^- \rightarrow Ag2O+H2O$$

4) The complex acicular nanocrystals of silver oxides of the present invention form neuronal three-dimensional superstructures (meso-crystals) by self-assembly of silver oxides containing silver peroxide.

Therefore, as shown in (FIGS. 8 and 9 in Patent Document 1), it is possible to form the meso-crystals from a Ag ion aqueous solution by performing a potentiostatic deposition using a Ag/AgCl electrode, or by oxidizing a quantum crystal of silver by alkali treatment, the meso-crystal had better be formed from the silver complex quantum crystals, for example, silver thiosulfate quantum crystals by alkali treatment (treatment with sodium hypochlorite aqueous solution).

5) As long as the disease-related substance can be adsorbed, it is possible to use the plasmonic chip disclosed (Patent Document 2) in which a thin film of silver and zinc oxide is formed on a substrate having a periodic structure with a pitch of 350nm which realizes high detection, sensitivity, and rapidity of a fluorescent labeling marker, or a localized plasmonic fluorescence enhancement sheet disclosed in (Patent Document 3) formed of silver nanoparticles having a uniform particle system by dispersing the thin film of silver and zinc oxide in an organic solvent and self-assembling the metal nanoparticles in a two-dimensional direction by volatilizing the organic solvent.

"Sample for Use in the Invention"

A sample is prepared from a body fluid which contains blood. Because red blood cells exhibit strong autofluorescence, it is better to centrifuge to get only plasma from blood. In the case of cancer disease as a disease-related substance, a dilution rate is determined to facilitate measurement of autofluorescence of fragmented DNA (subdivided nucleosomes) by 10 to 50-fold dilution. Among them, 20 to 30-fold dilution is preferrable for silver oxide meso-crystals formed by alkali treatment of silver thiosulfate complex quantum crystals prepared by dropwise addition of about 1000ppm of aqueous silver thiosulfate complex on phosphor bronze. In the case of cells, it is better than a mechanical disruption because it does not alter their physical properties. Advantageously, fragmented DNAs (fragmented nucleosomes) are stable protein conjugates (Protein-bound DNA fragments: nucleosome or chromatin) to form and exhibit relatively strong positive charges. Therefore, since it is selectively captured in plasmonic metal meso-crystals which exhibit a negative charge and exhibit a surface plasmon enhancement effect by the excitation light, and moreover, the fragmented DNA (subdivided nucleosomes) is stable, it is possible to reproduce the characteristics of the autofluorescence of the protein binding body before drying, even when stored after drying in a vacuum or desiccant (silica gel) and redissolved in distilled water or the like. In desiccant (silica gel) drying, this realizes the detection, not only from on-site blood collection and collection of plasma by centrifugation, but also for drying in a container, as shown in FIG. 4, so that an examination request by mailing can be made. In addition, all diseases are often caused by the accumulation of abnormal proteins; for example, the common features of neurodegenerative diseases such as Alzheimer's disease, Parkinson's disease, amyotrophic lateral sclerosis, and Huntington's disease are due to the accumulation of aggregated abnormal proteins in neuronal cells. Abnormal proteins are cytotoxic and therefore cause neuronal degeneration and cell death. Although most aberrant proteins are marked by ubiquitination and degraded in the proteasome, the proteasome is unable to destroy aggregated aberrant proteins, and the accumulation of aggregates of ubiquitinated aberrant proteins in neuronal cells in neurodegenerative diseases suggests that the detection of aberrant proteins is possible using the Liquid Biopsy method for the detection of disease-related substances other than cancer. In addition, since the iPS cells may contain cancerous gene DNAs, the iPS cells can also be used as a sample to discriminate and remove canceration of iPS cells by the inventive autofluorescence method.

"Fluorescence Image Acquisition Process"

Here, the fragmented DNA (fragmented nucleosome) captured on the plasmonic metal meso-crystalline substrate is irradiated, and the autofluorescence of the captured fragmented DNA (fragmented nucleosome) is enhanced by the surface plasmon enhancement effect, and the fluorescent colonies are acquired as a fluorescent image.

As then excitation light, a laser light source of 4 05nm excitation light, which is said to be suitable for exciting hematoporphyrin derivatives (tumor affinity fluorescent substances) having different integration and excretion characteristics in normal and diseased tissues, was used. Protein conjugates were collected and centrifuged, and the resulting plasma was diluted 30-fold with distilled water for use.

c) "Fluorescent colony selection process"

With Olympus DM (Dichroic Mirror) 405-445/514
Since the result of FIG. 1 was obtained, for example, 10 points of high luminance for each sample (the number of points to be collected is determined according to the disease) were extracted, a circular ROI was created in the vicinity of the center, and spectral data was calculated. The 10-point adoption of fluorescent colonies is binarized with dedicated soft "cellSens", and fluorescent colonies with brightness above predetermined thresholds are adopted.
Other image acquisition conditions are as follows.

Laser :405nm 50%
Objective lens: 10 times (MPLFLN10×)
Pinhole diameter: 500 nm
Wave length: 460-504nm
slit width: 4nm
Step: 2 nm
Resolution: 1024× 1024 Averaging: 4 times

d) "Operation step"

The total area value of the pixels of the fluorescent colonies above the selected predetermined thresholds, or the ratio value by the total area ratio of the pixels in the two-wavelength region corresponding to RGB is measured.
As a result, Healthy subjects, Benign and malignant tumors can be distinguished from the total area value and the ratio value of the total area in different wavelength regions.
The ratio of the two wavelengths of R, G, and B and the ratio of the two wavelengths correlated therewith are used for identification of the healthy subjects, benign tumors and malignancies.
Images of 470-490nm and 600-620nm were acquired using an Olympus DM405-445/514, and the results of FIG. 2 were obtained as a result of checking whether there was a change in Ratio values. For diagnosis of prostate, G/R for benign tumor is around 2.0, healthy subjects is around 1.70, while malignancy is over 1.76 up to 1.86, which indicates a relationship with stage. As a result, the data accuracy can be improved by increasing the number of samples.
In addition, since the luminance differs for each point, the luminance at each wavelength was displayed at a ratio of 470nm as 100% luminance, and a graph was prepared with the average value of 10 points as data for each sample. Further, it was confirmed that a peak of a cancer-related substance was present at a wavelength of about 610nm as shown in FIG. 3.

"Self-fluorescence images of a target object and a capture target object observed by its fluorescence microscope"

The present invention is intended for detection of fragmented DNA (fragmented nucleosomes) that occurs abnormally in cancerous and other conditions. Protein conjugates associated with this type of disease preferentially trap in plasmonic metal meso-crystals because of their positive charge

It has been found that the emission of the excitation light is enhanced by the surface plasmon enhancement effect of the plasmon metal meso-crystal and emits autofluorescence having a luminance equal to or higher than a predetermined luminance which can be confirmed by a fluorescence microscope (FIG. 1). These protein conjugates are released by pathological cell death typified by apoptosis into blood and other body fluids by the occurrence of disease, and a plurality of colonies are observed like the constellation in the night sky shown in FIG. 1, and are observed as a spread of about 25$\mu$m in small ones and a spread of about 150$\mu$m in large ones. Because inhibitors of CAD (caspase-activating DNase) are degraded in apoptotic cells and activated CAD cleaves DNA in nucleosome units, they are captured as fragmented DNA (fragmented nucleosomes) that are roughly 200-bp-fold higher. In detail, cell-free DNA (cf DNA) in human plasma is released into the blood as a protein binding associated with histones or TFs, preferentially survives, and is derived primarily from myeloid lymphoid cell lineages in healthy individuals, but a contribution from one or more additional tissues has been considered in certain pathologies, revealing a nucleosome footprint (footprint) th at infers cell types from cfDNA in pathological conditions such as cancer and informs the origin of th at tissue (non-patent literature 5: Cell,2016January1 4; 164: Cell-free DNA comprises an in vivo nucleosome footprint that informs its tissues-of-origin). It has been reported that the tumor grade leads to a higher degree of necrosis and that tumor DNA (ct DNA) is increased in the circulation, that plasma DNA exhibits a predictable fragmentation pattern similar to nuclease-cleaved nucleosomes, that cf DNA size-distribution can be assessed in healthy individuals and cancer patients, and that cf DNA in plasma is also reported to be involved in tumourigenesis and metastatic progression, revealing the importance of cf DNA as a diagnostic biomarker for liquid biopsy (non-patent literature 4: Circulating Tumor DNA as a Liquid Biopsy for Cancer; Climinal Chemistry 2015; 61: 112-123).

Now, it is a widely accepted fact that the development of cancer is a genetic disease caused by abnormalities in oncogenes and tumor suppressor genes, and the sequence of nucleotides, it has become clear that not only genetic aberrations due to mutations and deletions, but also epigenetic genetic aberrations due to modifications to bases can lead to the development of cancer. The effects on this epigenetic gene are thought to primarily affect the transcriptional control mechanisms of the gene, including methylation modification of genomic DNA and acetylation and methylation modification of histone proteins in complex with genomic DNA.

Thus, the binding of this cf DNA to histone proteins is detected not only by genetic aberrations but also by detecting epigenetic genetic aberrations due to modifications not only to distinguish healthy individuals and cancer patients, but also to suggest the distinctiveness of the cancer site.

(Selective Capture of Cancer-Related Substances)

As a cancer-related substance in serum, the fragmented DNA (fragmented nucleosomes) contains histones (nucleosomes) in which DNA is wrapped, and chromatin (fibers) in a structure in which they gather and form a string. Globulins are also positively charged, but the increase is up to 2-fold or less compared to other cancer-related substances, whereas the substance detected in the present invention increases more than 100-fold or more as cancer progresses, suggesting that an increase other than globulins indicates that cancer-related substances are being detected, and that a total area of pixels above a certain threshold of the selected fluorescent colony is associated with the stage of cancer.

Embodiments of the present invention will be described in detail below with reference to the drawings.

Example 1

A 1000ppm aqueous solution of silver thiosulfate was prepared, and one drop of it was added dropwise onto a phosphor bronze plate.

When the solution was left to stand for about 3 minutes and blown off, a quantum crystal was produced which was confirmed by an SEM image. In a photograph (refer to FIG. 1 of Patent Document 1) showing various SEM images of the nanoparticle aggregates (quantum crystals) produced in Example 1, the crystal is a thin hexagonal prism of about 100nm, and has unevenness on the order of several nm appears on the surface. Facets peculiar to metal nanocrystals could not be confirmed. In a photograph (FIG. 6 in Patent Document 1) showing the relationship between the standing time and the quantum crystal shape after dropping on a phosphor bronze hill, first, a hexagonal quantum crystal. In the graph (FIG. 4 of Patent Document 1) showing the result of EDS spectrum (elemental analysis) of the quantum crystal, in which the crystal formed on the phosphor bronze plate had an element derived from silver and a complex ligand, when a 1000ppm aqueous solution of silver thiosulfate was prepared on the copper plate, one drop of the solution was dropped, left to stand for about 3 minutes, and the solution was blown off, only silver was detected and the complex ligand was not detected.

(Work of Quantum Crystals)

In the case of a 1000ppm silver thiosulfate complex aqueous solution, when dropped on a phosphor bronze plate and left for 3 minutes, it was confirmed from the SEM image that the quantum crystal was formed in a hexagonal prism shape of around 100nm, and that each hexagonal prism-shaped quantum crystal had roughness on the order of several nm, but facets peculiar to the metal nanocrystal could not be confirmed, and the elements derived from silver and complex ligands were detected by EDS elemental analysis, so it is presumed that the whole is a nanocrystal of a silver complex, and the roughness appearing on the surface is spread by forming quantum dots as clusters of silver in the complex. Looking at the phenomenon in which the silver complex quantum crystal is formed on the phosphor bronze plate of the present invention, while the nanoparticles of only silver are precipitated on the copper substrate, since only silver (electrode potential 0.80) is precipitated on the copper substrate because the equilibrium potential of the silver thiosulfate complex is 0.33, which is equivalent to the electrode potential of copper (0.34), and in the case of phosphor bronze, the electrode potential is 0.22, which is slightly lower, it is considered that the crystals of the silver complex are precipitated. Therefore, it seems important that 1) the complex aqueous solution is in the dilute region of 500-2000ppm, 2) the electrode potential of the supported metal is slightly base to the equilibrium potential of the metal complex aqueous solution, and 3) the metal complex is aggregated at the electrode potential difference in order to prepare the quantum crystal. In addition, the same result was obtained when an aqueous solution of 1000ppm thiourea silver complex was used.

(Consideration of Meso-crystals of Silver Oxide: No.1)

When a 5% sodium hypochlorite aqueous solution is dropped onto the quantum crystal substrate and the substrate is removed by treatment for 2 minutes, the crystal structure shown in FIG. 11 of Patent Document 1 is observed, and needle-shaped crystals and la are observed. Since rugby ball-shaped lumps and a large lump were observed, when the respective compositions are analyzed by EDS spectrum (elemental analysis), it is considered that the needle-shaped crystals from the following reaction equation are both composed of a composite crystal of silver chloride and silver oxide, because the result of FIG. 7 in Patent Document 1 shows no chlorine and therefore silver Ag and oxygen O are dominant.

$$Na_2S_2O_3 + 4N\ aClO + H2O \rightarrow Na_2SO_4 + H_2SO_4 + 4NaCl \quad\quad (1)$$

$$Ag^+ + NaCl \rightarrow AgCl + Na^+ \quad\quad (2)$$

$$Ag^+ + 3NaOCl \rightarrow 2AgCl + NaC103 + 2Na^+ \quad\quad (3)$$

$$Ag^+ + OH^- \rightarrow AgOH \quad\quad (4)$$

$$2Ag^+ + 2OH^- \rightarrow Ag_2O + H_2O \quad\quad (5)$$

Therefore, in the formation of meso-crystals according to the present invention, silver ions and thiosulfate ions are presumed to be caused by an alkali oxidation reaction in the presence of chloride ions, but only silver oxide is formed in an ordinary aqueous solution, but it is presumed that silver peroxide is dominantly formed from the following XPS measurement.

(Consideration of Silver Oxide Meso-crystals: No.2) XPS Measurements:

To the quantum crystal substrate, $25\mu l$ of a sodium hypochlorite aqueous solution was dropped for 2 minutes to form the meso-crystal substrate, and Ag and O were XPS-measured without etching the recrystallized substrate (model: ULVAC-FI Co., Ltd./PHI5000VersaProbeII (scanning X-ray photoelectron spectroscopy analyzer)). Further, for comparison of object, the Ag of the powder of silver oxide and the powder of silver chloride were measured. On the other hand, the recrystallized substrate was etched with an argon gas cluster ion gun for 5 minutes, and Ag and O were measured by XPS. The results of the XPS measurements (FIGS. 9 and 10 of Patent Document 1) and from the results of the EDS (FIG. 8 of Patent Document 1), it is recognized that the peak at around 529eV is an O peak derived from silver peroxide (AgO), and the peak at around 530eV is an O peak derived from silver oxide (Ag2O). When etched, the oxygen content decreases, but it can be said that the O peak derived from silver peroxide (AgO) at a peak around 529eV is larger than the O peak derived from silver oxide ($Ag_2O$) at a peak around 530eV, which indicates that silver peroxide is formed in the vicinity of the substrates. It is presumed that the catalytic action at the time of meso-crystal formation and the electrode potential of the substrate are influenced. Incidentally, EDS-measurement using the recrystallized substrate model: was performed by using JEOL Corporation/JSM-7001F (field emission analytical scanning electron microscopy).

Further, even when the quantum crystals of silver thiosulfate were treated with an aqueous solution of hypochlorous acid, an aqueous solution of 0.01 N caustic soda, an aqueous solution of 0.01 N hydrochloric acid, and an aqueous solution of 0.1 mol sodium carbonate, the similar results could not be obtained. Therefore, the formation of the needle-like crystals may be caused by the above reaction in the presence of silver ions and thiosulfate ions. Silver oxide is negatively charged in aqueous solution and may be reduced by light to precipitate metallic silver.

Since the tendency of silver peroxide is remarkable, it seems that it adsorbs positively charged cancer-related substances, and moreover, the surface plasmon enhancement effect between adsorbed cancer-related substances and silver particles can be obtained.

"Imaging of Fragmented DNA (Fragmented Nucleosomes)"

Nucleosomes are the basic blocks of chromatin, consisting of four histones (H2 A, H2B, H3, H4) wrapped around a histone octamer, but histones play an important part in regulating the accessibility of DNA as well as in the control of DNA. Post-translational modifications of histones control their interactions with DNA and other nuclear proteins, thus affecting reversible gene expression. Methylation, acetylation, phosphorylation, ubiquitination, SUMO, citrullination, and ADP-liposylation are the major types of histone modifications known. Depending on which sites in the histone sequence are subject to these modifications, surrounding gene expression can be activated or repressed. When various histone code-related antibodies (Genetex anti-histone antibodies) are used to examine the histone code hypothesis by observing the modified state of the histone code captured by the plasmonic chip on fluorescent images, it is possible to examine the histone code hypothesis.

It is believed that DNA methylation is deeply involved in this chromatin structure control. Genomic DNA sites with high levels of DNA methylation generally show robust chromatin organization, with transcriptional repression and reduced DNA mutation rates. There are also clear correlations between genomic D NA methylation patterns and genomic imprinting, X-chromosome inactivation, and cellular tumorigenesis. Therefore, the structural analysis of histones and chromatin in the fragmented DNA (fragmented nucleosomes) is a key to the association with cancer. So, analysis of factors that chemically modify histone tails has important implications. Using the plasmonic chip of the present invention, by trapping the fragmented DNA (fragmented nucleosome) as a cancer-related substance, the presence or absence of the cancer symptom can be judged by the fluorescence imaging diagnosis by the amount of the fragmented DNA captured on the crystal. Then, it is possible to determine the extent to which the cancer symptom is, the cancer comes from which organ and the progression state is by analyzing chemical modification and remodeling factor of the histone tail. Therefore, if a laser beam with a wavelength of 445nm is used as the spectroscopic condition, and the "BS10/90" manufactured by Miller Olympus for Laser is used, and the spectroscopic spectrum is measured in 5nm increments in the range of 455 to 655 nm, a peak is observed in the vicinity of 515nm in the sample collected from the blood of a colorectal cancer patient, and the peak cannot be observed in the sample collected from the blood of a healthy person, and it is considered that it is possible to determine which organ cancer is detected by spectroscopy alone, or by considering the total area of pixels with a predetermined threshold or more in the RGB region, or the ratio G/G,G/B of the total areas of pixels with a predetermined threshold or more in the two wavelength regions in the RGB region. Then, information on how cancer develops and evolves through this chromatin remodeling event allows clinicians to more accurately predict how such cancers are likely to respond to specific chemotherapeutic agents, and in this way, chemotherapy based on knowledge of tumor chemosensitivity can be rationally designed.

In the present embodiment, Ratio value of the total area ratio of the pixels equal to or larger than the predetermined thresholds in the two wavelength regions is calculated from the fluorescence images obtained by R, G, and B, respectively, but the fluorescence images obtained by G tend to have a high level of benign tumors, and the fluorescence images obtained by B tend to have a high level of malignant tumors. Therefore, in the examples, analysis of Ratio values by G/R for prostates resulted in a range of around 2.0 for benign tumors, 1.7 for healthy subjects, and 1.76 to 1.86 for malignancies, whereas analysis of Ratio values by B/G for colon resulted in a range of 1.9 to 2.0 for malignancies, a range of 0.2 to 0.8 in healthy subjects and around 0.1 in benign tumors.

(G region short wavelength region/G region long wavelength region pixel ratio)

Fluorescence images were obtained by limiting the wavelength of fluorescence to be measured using an LED-light source (XYLIS wavelength: 360-770nm) with the following fluorescence wavelength filters on the Olympus erect microscopy BX-63. By adopting pixels above a predetermined threshold in each wavelength region

The total area is adopted in the same manner as described above. A sample of nine colon examination patients (plasma obtained by centrifugation from blood) was dropped onto the above-mentioned biochip to prepare a sample, and the total area of pixels in the G region on the molecular side and the G region on the denominator side over a predetermined threshold was obtained. BP470 ~495nm was used as a filter for molecular-side excitation light, and BA510 ~55 0nm was used as a fluorescent filter. Pixels with thresholds greater than or equal to a predetermined value are selected from the fluorescent images obtained by using the mirror unit, and the total area of the pixels is calculated as Ratio molecules.

On the other hand, BP460 ~ 480nm was used as a filter for exciting light on the denominator side, and BA495 ~ 540nm was used as a fluorescent filter. Pixels having thresholds equal to or higher than a predetermined value are selected from the fluorescent images obtained by using the mirror unit, and the total area of the pixels is calculated as the denominator of Rati o. Ratio of the G-region numerator/G-region denominator was calculated and compared with the stages determined by separate examination, as shown in Table 1 below. Thus, it was found that the results obtained by the method of the present invention were closely related to the malignancy/benign property and stage determined by the actual examination.

| Type | Specimen No. | Malignant/Benign | Ratio | Stage |
|---|---|---|---|---|
| Colon specimen | No.35 | Malignant | 2.13 | • (unknown) |
| | No.2 | Malignant | 2.06 | • |
| | No.22 | Malignant | 1.87 | • |
| | No.6 | Malignant | 1.76 | • |
| | No.8 | Malignant | 1.18 | • |
| | No.12 | Malignant | 1.18 | • |
| | No.25 | Benign | 0.91 | |
| | No.44 | Benign | 0.90 | |
| | No.37 | Benign | 0.84 | |

## Claims

1. An auto-fluorescent liquid biopsy which comprises:

    a) a step of charge-capturing a fragmented DNA (fragmented nucleosome) as a disease-related substance in a sample comprising a body liquid or a culture liquid containing cells by using a measurement substrate having a plasmon metal meso-crystalline region;
    b) a step of irradiating excitation light to the captured fragmented DNA (fragmented nucleosome) on the plasmon metal meso-crystal, enhancing its autofluorescence by a surface plasmon enhancement effect, determining a fluorescence image of a fluorescence colony of the fragmented DNA (fragmented nucleosome) by determining a certain measurement region (ROI), and acquiring a fluorescence colony image of any region of RGB;
    c) a step of adopting a pixel exhibiting a luminance equal to or higher than a predetermined threshold of the fluorescence colony image;
    d) a step of calculating 1) a total area value of pixels above a predetermined threshold in two different wavelength regions of an adopted measurement region, or 2) a ratio of a total area value of pixels above a predetermined threshold in a predetermined wavelength region of an adopted measurement region.

2. The Liquid Biopsy method of claim 1, wherein the plasmonic metal meso-crystals comprise silver peroxide meso-crystals, wherein the plasmonic metal meso-crystals have a positive charge and a surface plasmon enhancement effect.

3. The Liquid Biopsy method of claim 1, wherein the step of exciting the captured fragmented DNAs (fragmented nucleosomes) is carried out by excitation wavelengths in the same or different two wavelength regions of the B or G regions to obtain fluorescent colonies, the step of collecting fluorescent colonies is carried out through filters in different two wavelength regions, and wherein the total area value of pixels in the B region above a predetermined threshold is adopted as a malignant signal, while the total area value of pixels in the G region above a predetermined threshold is adopted as a benign signal, and the ratio value of the B region/G region or the G region/B region of the total area value of pixels collected therefrom is adopted as a final decision.

4. The method of claim 2, wherein the B/G ratio range from 1.9 to 2.0 is adopted for malignancies, and the B/G ratio range from 0.2 to 0.8 is adopted for healthy individuals, the B/G ratio of about 0.1 is adopted for benign.

5. The method of claim 1, wherein the captured fragmented DNA (fragmented nucleosome) comprises a methylated cancer suppressor gene, a hypermethylated RB gene, and p16 in addition to p14, p53, etc.

Fig.1

Spectral data

Image acquisition conditions

Laser 405nm 50%

Objective 10× (MPLFLN10×)

Pinhole diameter 500nm

Acquired wavelength 460 nm-504 nm

Slit width 4nm

Step 2nm

Resolution 1024×1024

Averaging 4 times

Setting the analysis ROI

Extract 10 points with high brightness for each sample

Create a circular ROI near the center and calculate spectral data.

Fig.2

Ratio data

Image acquisition conditions

Laser 405nm 50%

Objective 10 × (MPLFLN10 ×)

Pinhole diameter 500nm

Wavelength Acquired 470 nm–490 nm, 600–610nm

Resolution 1024 × 1024

Averaging 4 times

Setting the analysis ROI
Extract 10 points with high brightness for each sample
Create circular ROIs near the center and calculate the mean Ratio.

Fig.3

## Ratio data

Spectroscopic spectra were obtained using BS/10/90, and a peak was confirmed at around 610nm. However, analysis based on spectral data cannot be performed up to around 500nm because of the effect of reflected light.

To this end, DM405-445/514 was used to acquire 470-490nm and 600-620nm images to determine if there was a change in Ratio values.

Fig.4

(a)                                    (b)

Lid

Silica gel

Plasma

Container

Plasma
desiccant

## Fig.5A

### 1.Method of dropping the sample onto the proteo chip

● Dilute serum samples

10ul of a 30-fold dilution of serum with H2O is added dropwise

● Drop 10ul of the diluted sample onto the proteo chip.

10 ul drop

Silver peroxide
mesocrystal

● After dropping the sample, allow to stand for 3 minutes.

● After 3 min, wash the diluted sample with H2O.

Cancer-Related Substances Adhered to Proteo Chip Remaining

Enlarged view

Silver peroxide mesocrystal

Cancer-related substances

Quantify the fluorescent area

# Fig. 5B

## 2. Analysis range of the proteo chip

Enlarged view

Silver peroxide mesocrystal

Cancer-related substances

Fluorescence images are taken BZ-X710 fluorescence microscopy (detailed in *4).
Setting the Analysis Range of Fluorescence Images (5mm Diameter) on the Software

Fluorescence image

Silver peroxide mesocrystal

About 8mm in diameter

Analysis range (within red dotted line)

5mm in diameter

# Fig. 5C

### 3. To quantify the area of the proteo chip

Substances with strong fluorescence within the analysis range are selected from the fluorescence image on the software (substances with a threshold value of 13 or more), and the area value is calculated.

Fluorescence image

Silver peroxide mesocrystal

About 8mm in diameter

Analysis range (within red dotted line)

5mm in diameter

Highly fluorescent material

Calculate the area value of the substance with strong fluorescence (blue deposit, threshold value 13 or more) (Units: um2)

From the area value
A   Low Cancer Risk 0-19999
B   Observation required 20000-29999
C   Cancer Risk 30000-
Classify into three categories

Fig. 6

EP 4 083 223 A1

# CellSens analysis procedures

(1)Obtaining RGB3 fluorescent images per sample

(2)Determine the scope of analysis (enclosed by ROI)

(3)Debris excluded by determining analysis conditions (range of circularity and luminance)

(4)To quantify each RGB

(5)A Ratio of B/G, G/R, etc. is prepared from the numerical value of the total area of RGB obtained.

B/G Ratio levels of 1.9-1.0 for malignant tumors
        Benign tumors around 0.1 (due to high G)
        From 0.2 to 0.8 in healthy subjects

Scope of malignancy because (e.g.) is 1.75

※Analysis conditions differ by RGBdata.

B Luminance 28000 to 50000 Circularity 0.3... Total area value 14000 (example)
G Luminance 27000 to 50000 Circularity 0.3... Total area value 8000 (example)
R Luminance 21000 to 50000 Circularity 0.3... Total area value 4000 (example)

Fig. 7

Fig. 8

●G on the molecular side

Excitation filter
(470-495nm)

Fluorescent filter
(510-550nm)

Mirror unit

Fluorescence image

Numerator values
of Ratio area of the
fluorescent images
using the filters
described above

Numeric value of
the denominator of
Ratio of the mth
fluorescent pictures
using the above
filters

●G on the denominator side

Excitation filter
(460-480nm)

Fluorescent
filter
(495-540nm)

Mirror unit

Fluorescence image

EP 4 083 223 A1

In this way, two images of two wavelengths are measured from one tip, and a numerical value called a Ratio value is obtained from the analyzed ratio.

1. Autofluorescence Measured with Specified Two Wavelengths of Light

- Measure images for two wavelengths with one sample (one Proteo tip)
- Chip deposits emit autofluorescence at each wavelength

Wavelength of the identified malignancy

Malignant wavelength range B

Wavelength of the benign tumor identified

Benign wavelength range G

2. Selecting and quantifying materials with high brightness

- Select a material with high brightness from the measured image and calculate the area value.

Wavelength of the identified malignancy

Select in the malignant wavelength range

- Calculate area values for all selected substances e.g., 10000

Select in the benign wavelength range

- Calculate area values for all selected substances e.g., 2000

Wavelength of the benign tumor identified

3. Calculate Ratio values

Calculate Ratio value from the ratio of the calculated area value.

Example
Malignant wavelength/benign wavelength
B/G

10000/2000
= 5.00(Ratio)

Fig. 9

EP 4 083 223 A1

Fig. 10

Liquid Biopsy Identification

B/G ratio

Healthy person  Benign tumor  Malignancy

Fig. 11

## INTERNATIONAL SEARCH REPORT

| International application No. |
| --- |
| PCT/JP2020/048764 |

### A. CLASSIFICATION OF SUBJECT MATTER

C12Q 1/68(2018.01)i; G01N 33/483(2006.01)i; G01N 33/53(2006.01)i; G01N 33/543(2006.01)i; G01N 21/64(2006.01)i

FI:    C12Q1/68; G01N21/64 G; G01N33/543 595; G01N33/53 M; G01N33/543 575; G01N33/483 C

According to International Patent Classification (IPC) or to both national classification and IPC

### B. FIELDS SEARCHED

Minimum documentation searched (classification system followed by classification symbols)

C12Q1/68; G01N21/64; G01N33/483; G01N33/53; G01N33/543

Documentation searched other than minimum documentation to the extent that such documents are included in the fields searched

Published examined utility model applications of Japan    1922–1996
Published unexamined utility model applications of Japan    1971–2021
Registered utility model specifications of Japan    1996–2021
Published registered utility model applications of Japan    1994–2021

Electronic data base consulted during the international search (name of data base and, where practicable, search terms used)

JSTPlus/JMEDPlus/JST7580 (JDreamIII);
CAplus/REGISTRY/MEDLINE/EMBASE/BIOSIS/WPIDS (STN)

### C. DOCUMENTS CONSIDERED TO BE RELEVANT

| Category* | Citation of document, with indication, where appropriate, of the relevant passages | Relevant to claim No. |
| --- | --- | --- |
| Y | WO 2015/170711 A1 (MYTECH CO., LTD.) 12 November 2015 (2015-11-12) claims 2, 3, 5, paragraphs [0005]-[0007], [0020], [0022] | 1-5 |
| Y | JP 2019-513228 A (X-ZELL INC.) 23 May 2019 (2019-05-23) paragraphs [0107]-[0109] | 1-5 |

☐ Further documents are listed in the continuation of Box C.      ☒ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 09 February 2021 (09.02.2021) | 22 February 2021 (22.02.2021) |

| Name and mailing address of the ISA/ | Authorized officer |
| --- | --- |
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**
Information on patent family members

International application No.

PCT/JP2020/048764

| Patent Documents referred in the Report | Publication Date | Patent Family | Publication Date |
|---|---|---|---|
| WO 2015/170711 A1 | 12 Nov. 2015 | US 2017/0115216 A1 claims 1-3, paragraphs [0030], [0034] | |
| JP 2019-513228 A | 23 May 2019 | US 2019/0094115 A1 paragraphs [0169]-[0171] | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015170711 A
- JP 2011158369 A
- WO 2013039180 A

**Non-patent literature cited in the description**

- Detecting Peripheral Blood Circulation Tumor Cells (CTCs): Circulating tumor cell isolation and diagnostics: toward routine clinical use. *Cancer Res,* 2011, vol. 71, 5955-60
- **GORGES TM ; PANTEL K.** Circulating tumor cells as therapy-Related biomarkers in cancer patients. *Cancer Immunol Immunothe,* 2013, vol. 62, 931-939
- **PERMUTH-WEY J et al.** A Genome-Wide Investigation of MicroRNA Expression Identifies Biologically-Meaningful MicroRNAs That Distinguish between High-Risk and Low-Risk Intraductal Papillary Mucinous Neoplasms of the Pancreas. *PLoS One.,* 2015, vol. 10, e0116869
- **ELLEN HEITZER ; PETER ULZ ; JOCHEN B. GEIGL.** Circulating Tumor DNA as a Liquid Biopsy for Cancer. *Clinical Chemistry,* 2015, vol. 61, 112-123
- Cell-free D NA comprises an in vivo nucleosome footprint that informs its tissues-of-origin. *Cell,* 14 January 2016, 164
- *Current Status of Self-Fluorescent Endoscopes,* April 2016, vol. 58 (4
- *Biophysics,* 2013, vol. 53 (3), 166-169
- *Cell,* 01 January 2016, vol. 4, 164
- Circulating Tumor DNA as a Liquid Biopsy for Cancer. *Climinal Chemistry,* 2015, vol. 61, 112-123